# EUROPEAN PATENT SPECIFICATION

(11) **EP 3 512 574 B1**
(45) Date of publication and mention of the grant of the patent: **11.01.2023**
(21) Application number: 17734483.5
(22) Date of filing: 13.06.2017
(51) Int. Cl.: A61M 1/00, A61M 3/02, A61M 39/10, A61B 17/22

(54) **CONNECTOR FOR SURGICAL HANDPIECE**
VERBINDER FÜR EIN CHIRURGISCHES HANDSTÜCK
PIÈCE DE CONNEXION POUR MANCHE CHIRURGICAL.

(30) Priority: 15.09.2016 US 201662394994 P; 14.11.2016 US 201662421645 P
(43) Date of publication of application: 24.07.2019
(73) Proprietor: Integra LifeSciences Enterprises, LLLP, Princeton, NJ 08540 (US)
(72) Inventor: KETELHOHN, Robert A., Dunstable Massachusetts 01827 (US); COTTER, Daniel J., North Easton Massachusetts 02356 (US); MANANDHAR, Prakash, Lawrence Massachusetts 01841 (US); BERTORELLI, John, Andover Massachusetts 01810 (US); GUPTA, Saurav V., Medway Massachusetts 02053 (US); LEMIEUX, Erin-Anne A., Mont Vernon New Hampshire 03057 (US)
(74) Representative: Gill Jennings & Every LLP
(86) International application number: PCT/IB2017/053510
(87) International publication number: WO 2018/051196

(56) References cited:
- EP-A1- 1 607 075
- WO-A1-87/01926
- WO-A1-2004/045705
- US-A- 5 492 528

## Description

### BACKGROUND OF THE INVENTION

Embodiments of the present invention relate generally to connectors for surgical handpieces, for example, handpieces in ultrasonic surgical aspirator systems for tissue ablation.

Ultrasonic aspiration has become the standard of care for removal of tumors and diseased tissue in neurosurgery and general surgery. Ultrasonic aspirators are used for ultrasonic fragmentation of tissue at an operation site and aspiration of the tissue particles and fluid away from the site. Typically, ultrasonic surgical aspirators include an ultrasonic transducer supported within a handpiece, an ultrasonically vibrating horn or tip operably connected to the ultrasonic transducer, and a sleeve or flue positioned about the horn. The horn includes a longitudinally extending central bore having one end located adjacent a distal tip and a second end located adjacent the proximal end of the horn. The proximal end of the horn is adapted to engage a vacuum source to facilitate aspiration of fluid. The flue is positioned about the horn to define an annular passage. Irrigation fluid is supplied through the annular passage around the horn to the surgical site where it mixes with blood and tissue particles and is aspirated through the bore in the horn. By mixing the irrigation fluid with the blood and tissue particles, coagulation of the blood is slowed down and aspiration thereof is aided. When the longitudinally vibrating tip in such an aspirator is brought into contact with tissue, it gently, selectively, and precisely fragments and removes the tissue. U.S. Patent Nos. 5,015,227 and 4,988,334 disclose such ultrasonic surgical devices. A known ultrasonic aspirator on the market is the CUSA^{®} Excel Ultrasonic Surgical Aspirator (Integra Life Sciences Corporation, Plainsboro, New Jersey, U.S.A.).

Examples of existing handpieces include CUSA Excel 23 kHz and 36 kHz Handpieces (Integra Life Sciences Corporation, Plainsboro, New Jersey, U.S.A.) and those described in U.S. Patent Nos. 4,223,676, 4,425,115 and 6,214,017. Those existing handpieces require user installed 0-rings to provide sealing, support, and clasping functionality for the nosecone. The 0-rings need to be installed by the user with each surgical procedure. The 0-rings are small and difficult to install in operating room by practitioners wearing gloves, and the 0-rings can be placed incorrectly or on the wrong mating geometry. In addition, the 0-rings are different in diameter and could be selected and installed incorrectly. The 0-rings for different handpieces could be selected incorrectly in manufacturing and assembly. In other existing devices, 0-ring seals may be reusable and sterilizable but require periodic maintenance, and thus raise some concerns on ability to clean and sterilize over life.

Hence, those skilled in the art have recognized a need for a surgical handpiece connector with improved ease of use. Embodiments of the present invention fulfill this need and others.

US 5492528 A describes a surface-discriminating fragmenting handpiece.

### SUMMARY OF THE INVENTION

Briefly and in general terms, embodiments of the present invention provide a connector for surgical handpiece, such as a nosecone, that incorporates an overmolding technology. More specifically, the nosecone eliminates user installed 0-rings and enhances ease of use. The J-lock clasp of the nosecone and its seal is incorporated to a single proximal overmolded soft polymer and the distal diametrical seal is accomplished with a separate overmolded ring seal.

In accordance with a first embodiment of the invention, there is provided a connection apparatus for attachment of members of a medical device. The connection apparatus comprises a first member having a first body and a first connecting section, the first connecting section having a first end face and an inner surface; a second member having a second body, a second connecting section and a shoulder at the junction between the second body and the second connecting section, the second connecting section having a second end face and an outer surface shaped for telescoping into the inner surface of the first connecting section, the outer surface having a groove extending from the second end face towards the shoulder; wherein the first member has a nub on the inner surface extending radially inward for engaging the groove; and wherein the first member has an end overmold portion at least partially covering the first end face and positioned to bear against the shoulder, whereby the end overmold portion is compressed between the first body and the second body.

In more detailed aspects, in the connection apparatus, the first member has more than one nub on the inner surface arranged radially about the first connecting section, and the second member has more than one groove on the outer surface arranged radially about the second connecting section, and each nub is positioned to engage with each groove.

In accordance with a second embodiment of the present invention, a method for attaching members of a medical device is provided. The method comprises providing a first member having a first body and a first connecting section, the first connecting section having a first end face, an inner surface, a nub on the inner surface extending radially inward, and an end overmold portion at least partially covering the first end face; providing a second member having a second body, a second connecting section and a shoulder at the junction between the second body and the second connecting section, the second connecting section having a second end face and an outer surface, the outer surface having a groove extending from the second end face towards the shoulder; telescoping the second connecting section into the first connecting section; locating the groove in the outer surface of the second connecting section; positioning the nub to engage the groove; and moving the nub in the groove towards the shoulder until the end overmold portion contacts the shoulder whereby the end overmold portion is compressed between the first body and the second body.

Further, an ultrasonic aspirator apparatus for fragmenting tissue and removing fragmented tissue is provided, which does not form part of the claimed invention. The ultrasonic aspirator apparatus comprises a surgical handpiece comprising a housing, a nosecone attached to the housing, and a transducer mounted within the housing; a surgical tip connected to the transducer; an irrigation system connected to the handpiece for supplying irrigation fluid adjacent the surgical site for suspending fragmented tissue; and an aspirating system connected to the handpiece for aspirating fluid and tissue fragmented at the surgical site; wherein the nosecone has an inner surface and an outer surface, and has an internal overmold portion on the inner surface positioned radially about the nosecone to provide a fluid tight seal that prevents ingress of irrigation fluid into the housing. The ultrasonic aspirator apparatus may additionally comprise an end overmold portion in contact with the housing to provide a seal between the housing and the nosecone.

In further detailed aspects, the nosecone may have a plurality of recessed lobes on the outer surface, the lobes being circumferentially spaced about a longitudinal axis. For example, the nosecone may have a tri-lobe configuration on the outer surface to be grasped by a user.

Other features and advantages of the embodiments of the present invention will become more apparent from the following detailed description of the embodiments, when taken in conjunction with the accompanying exemplary drawings.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.

Embodiments of the present invention are described herein with reference to the drawings, in which:
FIG. 1 is a perspective view of an ultrasonic apparatus;
FIG. 2 is a perspective view of a handpiece with a nosecone in accordance with embodiments of the present invention;
FIG. 3 is a longitudinal-sectional view of a portion of the ultrasonic apparatus of FIG. 1;
FIG. 4 is a perspective view of an ultrasonic horn;
FIG. 5 is a perspective view of a nosecone fully assembled to a handpiece and supporting a flue (the flue tube is not shown in this drawing);
FIG. 6 is a perspective view of a nosecone in accordance with embodiments of the present invention;
FIG. 7 is a cross-sectional view taken along line A-A of FIG. 6.
FIG. 8 is a top plan view of the nosecone of FIG. 6;
FIG. 9 is a bottom plan view thereof;
FIG. 10 is a front elevational view thereof;
FIG. 11 is a distal end view thereof;
FIG. 12 is a proximal end view thereof;
FIG. 13 is a cross-sectional view taken along line B-B of FIG. 9;
FIG. 14 is a bottom plan view of the nosecone of FIG. 6 without the overmold portions;
FIG. 15 is a cross-sectional view taken along line E-E of FIG. 14
FIG. 16 is a cross-sectional view taken along line C-C of FIG. 15
FIG. 17 is a cross-sectional view taken along line D-D of FIG. 15
FIG. 18 shows the handpiece housing and nosecone in a dissembled state;
FIG. 19 is a perspective view of the handpiece housing;
FIG. 20 is a side view of the handpiece housing of FIG. 19;
FIG. 21 is an enlarged perspective view of the distal end of the handpiece housing of FIG. 19;
FIG. 22 is a cross-sectional view taken along line F-F of FIG. 20;
FIG. 23 is a cross-sectional view taken along line G-G of FIG. 20;
FIG. 24 is a partial longitudinal-sectional view of the handpiece housing and nosecone in an assembled state; and
FIG. 25 is a perspective view of a portion of the handpiece housing and nosecone in an assembled state.

### DETAILED DESCRIPTION OF THE PREFERRED EMBODIMENTS

Embodiments of the presently disclosed connectors for surgical handpieces will now be described in detail with reference to the drawings, in which like reference numerals designate identical or corresponding elements in each of the several views. As used herein, the term "distal" refers to that portion of the instrument, or component thereof which is farther from the user while the term "proximal" refers to that portion of the instrument or component thereof which is closer to the user during normal use. The terms "ultrasonic horn," "ultrasonic tip," "ultrasonic surgical tip," "surgical tip", "horn" and "tip" are used herein interchangeably.

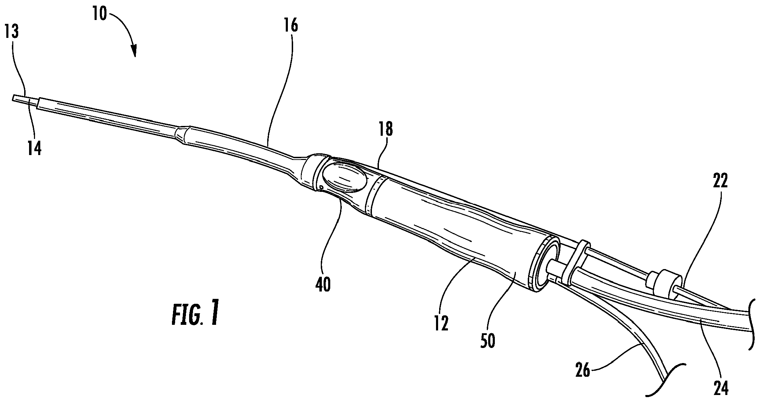

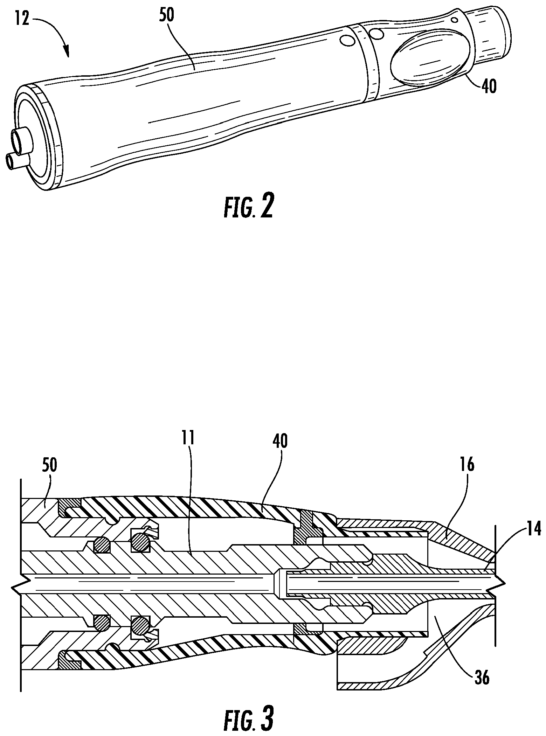

Referring now to FIGS. 1-3, one embodiment of the apparatus for ultrasonically fragmenting and aspirating tissue is shown. Generally an ultrasonic surgical apparatus 10 includes a handpiece 12 for use by a surgeon to direct fragmentation. The handpiece 12 encases a transducer (not shown) on which a surgical tip or ultrasonic horn 14 is fastened. The ultrasonic horn can be powered by the transducer and be ultrasonically actuated to fragment tissue and suction effluent via a central channel. A distal end portion 13 of the ultrasonic horn 14 extends beyond a distal end of a flue 16. Ultrasonic horn 14 is vibrated to fragment tissue during surgery. The ultrasonic horn may be made of titanium or other conventional materials known in the art.

A cooling and irrigation system which provides cooling fluid to the ultrasonic horn 14 is provided for maintaining temperature within an acceptable range. The handpiece 12 includes a housing 50, which may be formed of a sterilizable plastic, metal or other suitable materials or a combination thereof. The flue 16 provides a path for irrigation fluid or liquid and connects to the distal end of the handpiece 12. The flue 16 typically connects to the handpiece 12 via a nosecone 40. The flue 16 may include or attach to a flue tube 18. The nosecone 40 connects to the housing 50 and covers the internal ultrasonic horn 14.

An irrigation tube 22 connects to the flue tube 18 up-stream and supplies irrigation fluid through the flue tube 18 to an operative site during surgery. An aspiration tube 24 provides suction and a path for aspiration from the operative site to a collection canister (not shown). Alternatively, the aspiration tube may be mounted outside of the housing 50. An electrical cable 26 provides power to the apparatus or provides switching connections.

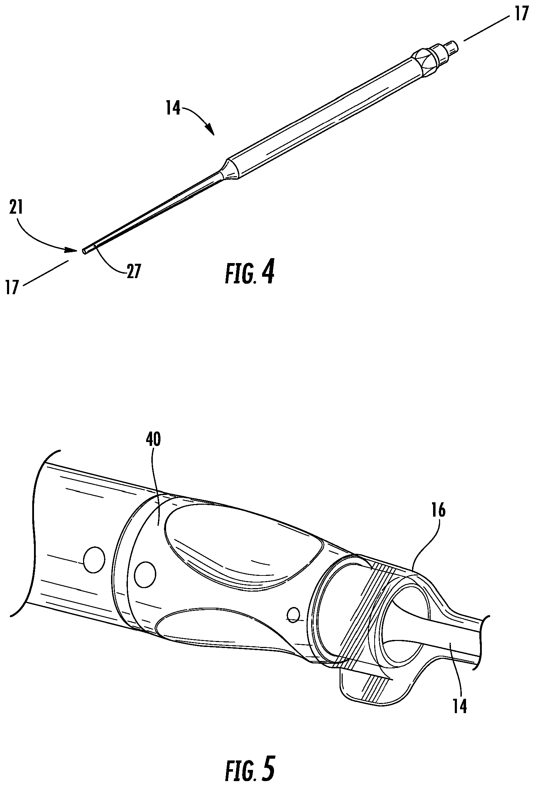

FIG. 4 illustrates an ultrasonic tip or ultrasonic horn 14, which is suitable for use with the above-described ultrasonic surgical apparatus for fragmenting and aspirating tissue. The ultrasonic horn has a throughbore 17 and a preaspiration hole or transverse bore 27. Although the ultrasonic horn as shown is a stepped horn, it is known that there are ultrasonic horns that are not stepped.

The ultrasonic horn 14 is substantially circular and disposed within the flue 16. During operation of the ultrasonic apparatus 10, irrigation fluid is supplied through the irrigation tube 22 and flue tube 18 into the flue 16. The flue 16 and the ultrasonic horn 14 define an annular cavity 36 therebetween. Irrigation fluid is supplied from flue 16 through the annular cavity 36 to the distal end of the ultrasonic horn 14. A transverse bore is formed in preaspiration holes 27 near the distal end of the ultrasonic horn 14 and communicates with the throughbore 17. The irrigation fluid is drawn from preaspiration holes 27 and the surgical site into an inlet 21 of the throughbore 17 along with fragmented tissue, blood, etc., and is removed from the surgical site via the throughbore 17 and the aspiration tube 24. The transverse bore provides an alternate route for fluid to enter throughbore 17 when inlet 21 becomes clogged. The nosecone 40 attaches to the housing 50 and covers internal portion of the ultrasonic horn 14.

In a more detailed aspect, irrigation liquid, for example saline, is necessary to cool the surgical tip and site of tissue fragmentation. This irrigation liquid may be provided to the flue with a peristaltic pump at a rate as low as 2 to 3 ml/min, which is only typically about a drip or two a second. The irrigation liquid is supplied at the proximal end of the ultrasonic horn. The irrigation liquid progresses to near the distal end of the ultrasonic horn, where two preaspiration holes, which may each have a 0.015 inch diameter for example, suction a majority, perhaps 90-95%, of the irrigation through the holes connecting the outside horn diameter to the central suction channel. This action of irrigation and suction supports a contiguous cooling circuit for the vibrating titanium metal and it also helps to wet effluent such as blood and tissue in the central channel. Some irrigation is also favorable to cooling the surgical site, improving coupling to tissue, and affording cavitation necessary to emulsification and aspiration of tissue, such as tumors.

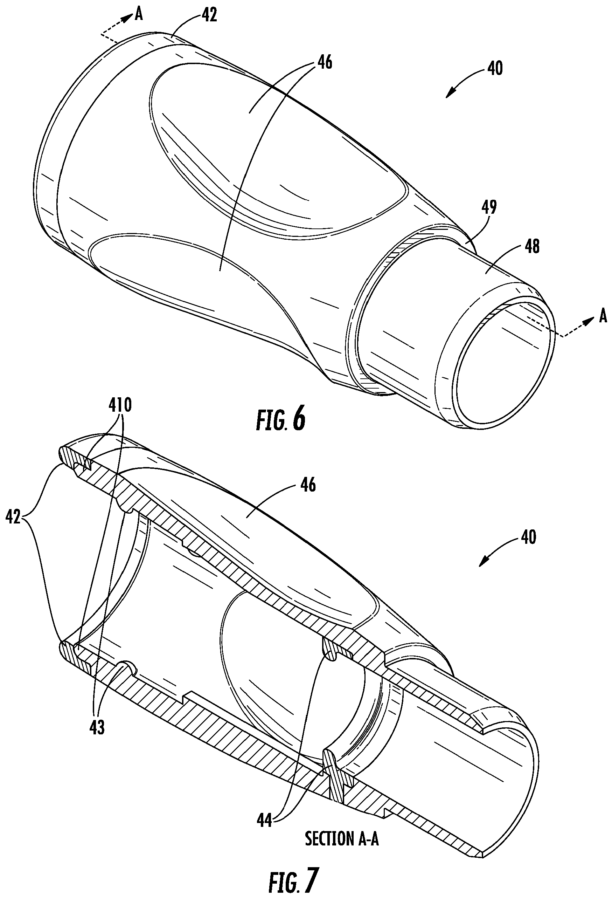

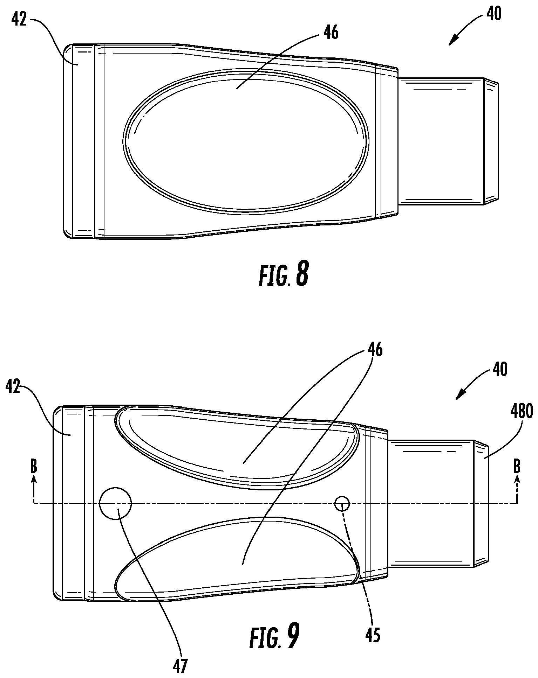

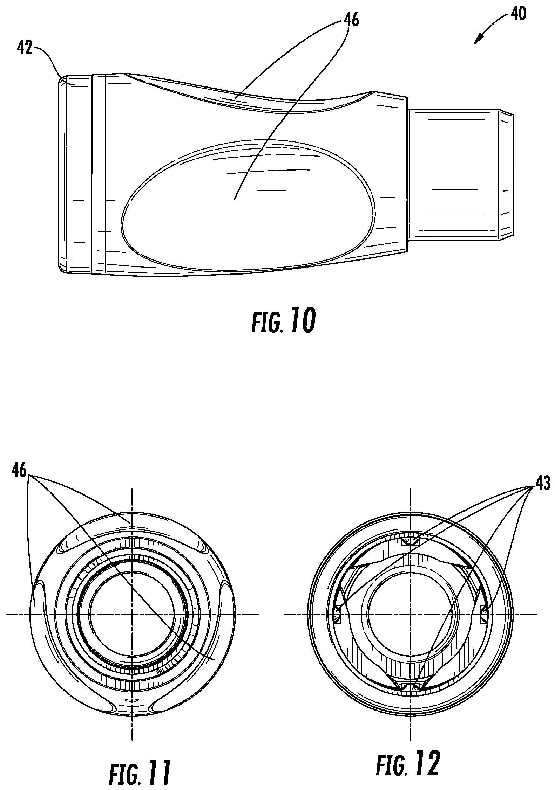

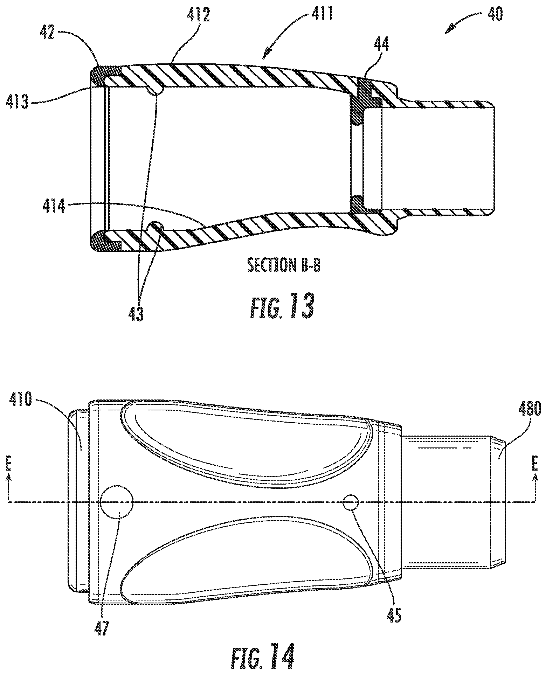

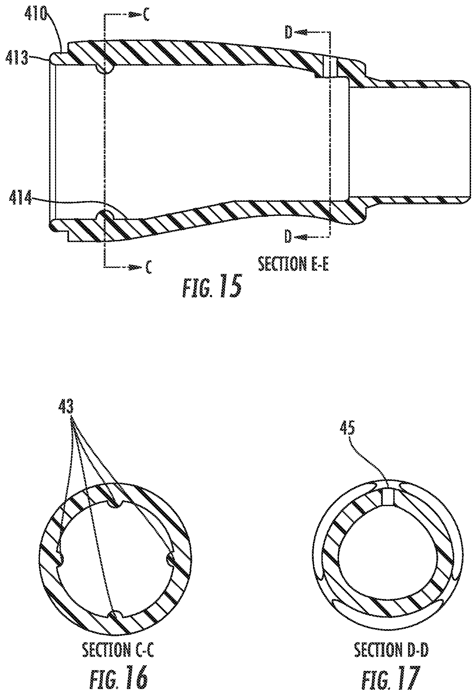

The nosecone 40 will be described in more detail with reference to FIGS. 6-17. The form of the nosecone is generally a cylindrical taper having a generally circular cross-section and a neck 48 at the narrower end of the taper. The tapered nosecone body transitions to the neck 48 through a nosecone shoulder 49. The neck 48 has a constant diameter that is smaller than the diameter of the narrower end of the taper. The neck 48 may have a beveled end 480. It provides a surface area for holding the flue 16. Alternatively, the nosecone may be substantially cylindrical and may have a cross-section that is generally oval or of another suitable shape.

The nosecone 40 may have a plurality of recessed lobes 46 on the outer surface. The lobes are circumferentially spaced, symmetrically or asymmetrically, about a longitudinal axis. The recessed lobes 46 may each have a recessed area of a shape suitable to be grasped by a user with fingers. For example, the recessed area may be oval or circular. In an exemplary embodiment, along the tapered section, three lobes, 120 degrees apart, provide finger grip placement. The tri-lobe configuration on the outer surface of the nosecone provides an ergonomic hand grip area that makes it easy for a user to grasp and hold the handpiece in hand and thus enhances comfort for the user.

An internal overmold portion 44, located proximal to the neck 48, serves as a primary fluid seal. The overmold portions eliminate the need for O-rings and thus enhance ease of assembly. The overmolding may be made from a medically comparable thermoplastic elastomer, for example polypropylene or other conventional materials used in gaskets, stoppers and seals as known in the art. A thermoplastic elastomer is a plastic polymeric material that becomes pliable or moldable above a specific temperature and solidifies upon cooling. The materials of the nosecone body and overmold portions are selected for adequate adherence and withstand of sterilization. Once fully assembled, the internal overmold portion 44 of the nosecone 40 conforms to the internal ultrasonic horn 14 to prevent fluid ingress into the handpiece 12.

The nosecone 40 has an end overmold portion 42 at the large diameter open end. The end overmold portion 42 is a part of the coupling mechanism that affixes the nosecone 40 to the housing 50. The coupling mechanism can be used not only for connecting the housing and nosecone components of surgical handpieces, but also for attachment of members of other medical devices or other apparatus.

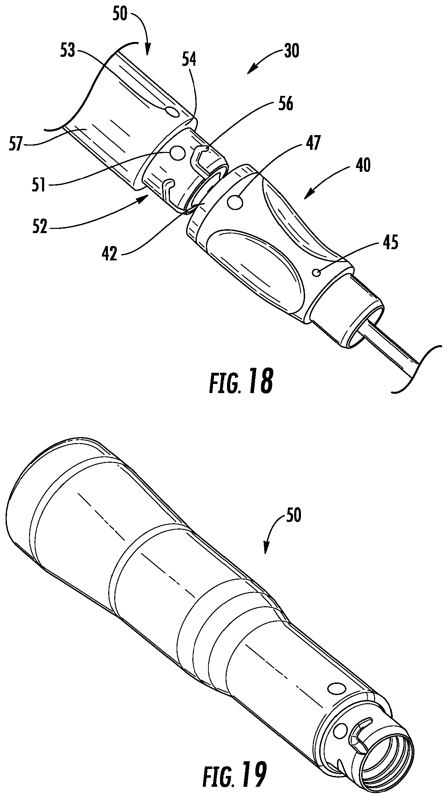

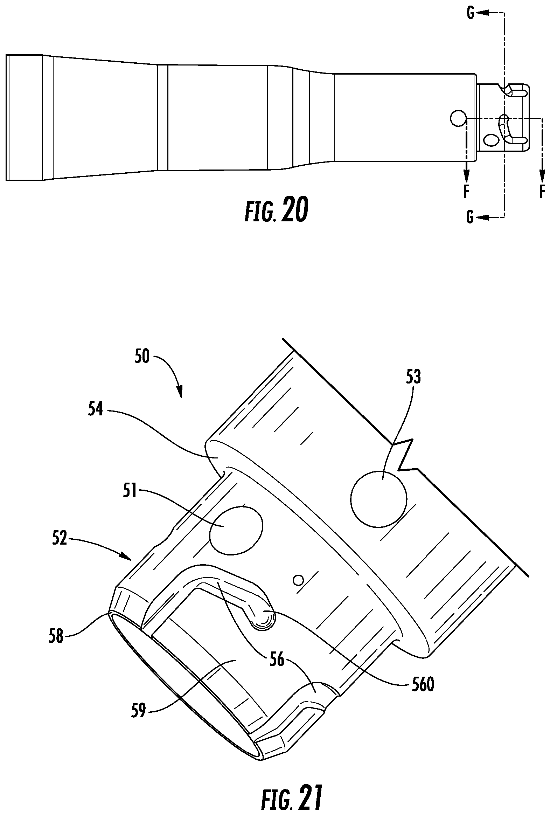

Turning now to FIGS. 18-23, a connection apparatus 30 for attachment of members of a medical device is provided. The connection apparatus 30 comprises a first member, for example, a nosecone 40, and a second member, for example, a housing 50. The first member or nosecone 40 has a first body 411 and a first connecting section 412. The first member or nosecone may further comprise a lip 410 extending from the first connecting section (or nosecone connecting section) 412. The first connecting section has a first end face 413 and an inner surface 414. The second member or housing 50 has a second body 57, a second connecting section (or housing connecting section) 52 and a shoulder 54 at the junction between the second body 57 and the second connecting section 52. The second connecting section has a second end face 58 and an outer surface 59 shaped for telescoping into the inner surface of the first connecting section 412. The outer surface 59 comprises a groove 56 extending from the second end face 58 towards the shoulder 54. The groove 56 may be a curved or angled groove, such as a J-shaped groove, forming a moving track for the nub 43, with a rest position 560 at the end of the track, located between the shoulder 54 and the second end face 58. The first member or nosecone 40 comprises a nub 43 on the inner surface 414 extending radially inward for engaging the groove 56. The first member or nosecone 40 has an end overmold portion 42 at least partially covering the first end face and positioned to bear against the shoulder 54, whereby the end overmold portion 42 is compressed between the first body 411 and the second body 57.

The J-shaped groove may comprise a plurality of segments. For example, it may have three segments. The first segment extends from the second end face of the housing towards the shoulder of the housing and is generally perpendicular to the second end face. The second segment is at an angle relative to the first segment, for example, at an angle of about 15 to about 75 degrees, and preferably between about 30 to about 60 degrees. The third segment constitutes the rest position, which is shorter than the first segment and the second segment. It may be at an angle relative to the second segment. It is contemplated that the rest position may simply be the end of the second segment without being at any apparent angle relative to the second segment.

The end overmold portion 42 may be present on the outer diameter or inner diameter of the nosecone connecting section or both diameters. If a lip 410 exists on the nosecone, the end overmold portion may be present on the outer diameter of outer diameter or inner diameter of the lip 410 or both diameters. In the exemplary embodiment as shown in FIG. 7, the end overmold portion 42 is positioned at least partially on the outer diameter of the lip 410 and flush with the outer surface of the nosecone 40.

The first member 40 may have more than one nub on the inner surface 414, arranged radially about the first connecting section 412, and the second member 50 may have more than one groove 56 on the outer surface arranged radially about the second connecting section 52. The nubs 43 may be spaced symmetrically or asymmetrically apart, and the grooves are spaced accordingly such that each nub is positioned to engage with a respective groove. In the exemplary embodiment as shown, the first member 40 has four nubs 43 on its inner surface evenly spaced approximately 90 degrees apart, and the second member 50 has four grooves 56 on its outer surface evenly spaced approximately 90 degrees apart.

In the embodiment as shown, the first member is a nosecone and the second member is a surgical handpiece housing. It is contemplated that the first member may be a surgical handpiece housing and the second member may be a nosecone.

In operation, a user positions each nub 43 to engage a respective groove 56 and moves each nub 43 in the groove 56 towards the shoulder 54 of the housing 50 until the end overmold portion 42 contacts the shoulder 54. The grooves may be generally evenly spaced apart on the housing about a longitudinal axis. Likewise, the nubs may be generally evenly spaced apart on the inner surface of the nosecone about a longitudinal axis accordingly for engaging the grooves on the housing.

The inside of the nosecone is hollow to provide clearance space in order for it to slip over the ultrasonic horn 14 and connect to the housing 50. In the exemplary embodiment as shown in the drawings, four nubs 43, in the form of spheres that are about 0.06 inches in diameter, form part of the coupling mechanism that affixes the nosecone 40 to the housing 50. The spheres may have a diameter in the range of about 0.01 to about 0.10 inches, for example, in the range of about 0.2 to about 0.8 inches. The nubs may be in the form of other raised structures suitable for engaging with the grooves.

The overmold portions are preferably made of a material that has elastomeric properties of having a low Young's Modulus and flexibility. A proper material should also be able to achieve a strong, stable bond to the substrate. It also needs to be able to be injected into a mold in a semi-fluid or fluid state at an elevated temperature and to remain on the substrate and retain its strength and provides elasticity after it has cooled and solidified. In a preferred embodiment, the body of the nosecone is made from glass-filled polypropylene (PP) which has appropriate strength, stiffness, melting temperature and ability to adhere with overmold material (thermoplastic elastomer). In another preferred embodiment, the overmolding is made from a healthcare thermoplastic elastomer grade used in gaskets, stoppers and seals. It has the ability to bond to a variety of substrates, for example PP. Compression set, reseal capability, and an ability to be sterilized under autoclave and ethylene oxide are some of the factors to be considered in selecting materials for the design.

There are several identification markers on the apparatus for the coupling mechanism. The housing has a housing identification marker 53 on the housing body 57 and another connection identification marker 51 on the housing connecting section 52. The nosecone 40 has a nosecone identification marker 47. The identification markers may be in any form, shape and color as desired, such as white dots.

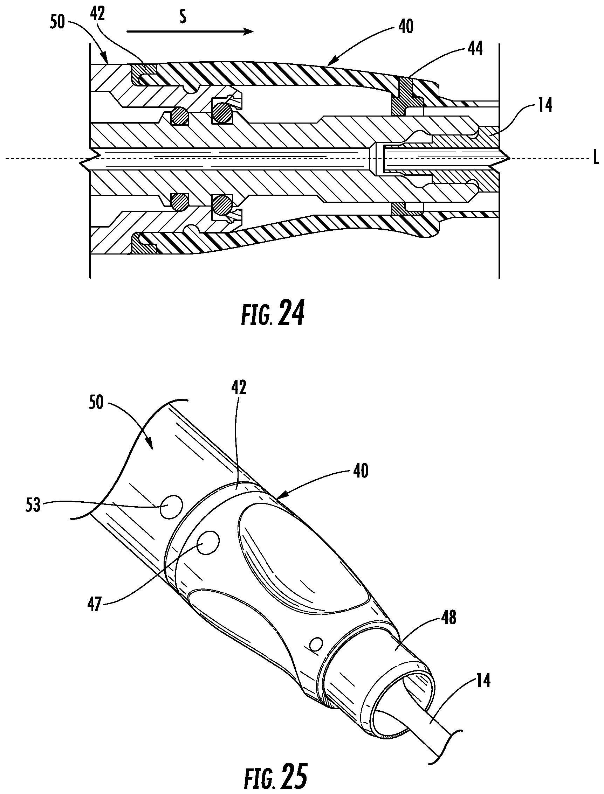

Referring now to FIGS. 24 and 25, attaching the nosecone in some embodiments of the present invention is simple because the user only needs to install one component without the need for handling separate O-rings. Once an ultrasonic horn 14 has been torqued to a handpiece 12, the nosecone 40 can be slipped over the ultrasonic horn 14. As the nosecone approaches the housing connecting section 52 of the housing 50, the nosecone identification marker 47 on the nosecone 40 should be oriented to align with the connection identification marker 51 on the housing connecting section 52 of the housing 50. This essentially guides the nosecone nubs 43 to the grooves 56 in the housing 50.

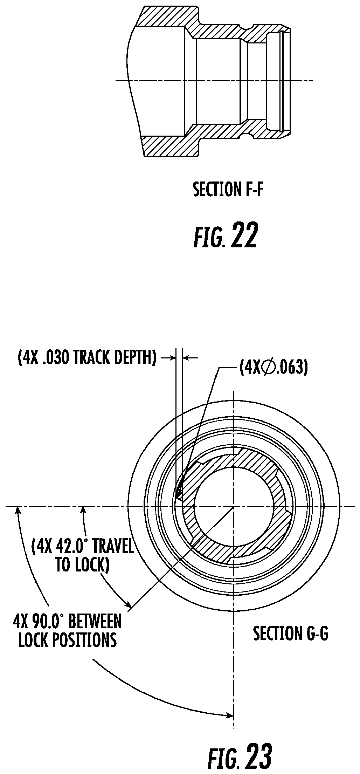

Once the nub 43 has joined the groove 56 of the housing 50, it will follow along and rotate clockwise through the J-shaped track until it reaches the rest position 560, where the end overmold portion 42 compresses. Depending on the orientation of the grooves, the nosecone may travel for a degree to lock. In the exemplary embodiment as shown in FIG. 23, the nosecone travel for about 42 degrees in the grooves to lock.

The nosecone 40 remains fixed in the rest position 560 by means of the expanding of the end overmold portion 42. As shown by arrow S, the end overmold portion 42 acts as a spring, applying a linear force to drive the nub 43 against the end of the groove 56. Locking of nosecone 40 is achieved via elastomer expansion that pushes the nub 43 to contact the wall of the groove 56, for example, the wall in the rest position 560. The flexible property of the thermoplastic material of the end overmold portion 42 works like a spring, expanding out in the longitudinal axis L which results in a linear force to drive the nosecone nubs 43 against the wall at the end of the grooves 56 of the handpiece housing 50 to secure the nosecone firmly to the handpiece.

Although preferably the end overmold portion 42 is an annular piece, it does not have to be a complete annulus. It may have one or more gaps in the ring-shaped structure so long as the end overmold portion provides a spring force that allows locking of the housing 50 and the nosecone 40.

FIG. 25 shows alignment of the housing identification marker 53 on the housing 50 and the nosecone identification marker 47 on the nosecone 40 in fully coupled state. The coupling mechanism is verified to the user via the alignment of the nosecone identification marker 47 to the housing identification marker 53. Once fully assembled, the internal overmold portion 44 conforms to the internal horn 11 and forms a fluid seal to prevent fluid ingress into the handpiece 12.

The internal overmold portion 44 on the inner surface of the nosecone 42 is positioned radially about the nosecone 42 to provide a fluid tight seal that prevents ingress of irrigation fluid into the housing. In addition, the internal overmold portion 44 enables keeping the flue primed, as the surgeon puts the handpiece down on the patient or a table during surgery. The inner seal helps keep the flue primed or irrigation functional immediately, when the handpiece is picked back up by the user. The inner seal reduces the volume that the trickle of irrigation could fill if the system were laid down with the tip up slightly. The inner seal helps keep the liquid in the flue to tip space until use.

An insert molding method can be used for manufacturing the nosecone in some embodiments of the present invention. In the manufacturing method, a premolded insert is placed into the mold before a thermoplastic material is shot. After the mold assembly is in place, the overmold material is then injected into the mold, either in a semi-fluid or fluid state, using conventional methods known in the art. For making the internal overmold portion, the thermoplastic material can be injected through an opening 45. The manufacturing of the nosecone could be highly economical, such that a mold of the nosecone matrix or substrate is followed by overmolds in a single tool with two or more actuations as needed. The nosecone with overmold portions could be supplied as a disposable, greatly enhancing ease-of-use with simple assembly in the operating room.

Although the exemplary embodiments of the present invention show the end overmold portion and the internal overmold portion as two separate elements, it is contemplated that the two elements may be two portions of a single piece. The nosecone may have a single overmolded piece that includes an end overmold portion, an internal overmold portion, and a connecting overmold portion that connects the end and internal overmold portions. The connecting overmold portion may be on the inner surface or the outer surface of the nosecone. For example, the nosecone may have an elastomer encased body as a single piece which comprises an end overmold portion, an internal overmold portion, and an elastomer wall on the outer surface of the nosecone. In addition, the elastomer may form part of the nosecone body.

Although one embodiment of the present invention is illustrated with a J-lock clasp in the handpiece, it is contemplated that the end overmold portions can work with other connecting, coupling or interlocking mechanisms in which O-rings or gaskets are used or desired, such as locking teeth, snap-ears, other snap-in locking features, and other mechanisms. The spring force or seal function necessary to such a connecting, coupling or interlocking mechanism can be achieved through applying an overmolding technology as described in the embodiments of the present invention.

The connection apparatus embodiments of the present invention is useful for attachment of components in machines, apparatuses, instruments and devices including but not limited to medical devices. The nosecone embodiments of the present invention can be applied to various surgical handpieces, such as CUSA Excel 36 kHz and 23 kHz ultrasonic handpieces and other surgical handpieces. The overmolding technology can be applied on longer angle adapted nosecones as well including longer shroud nosecones with angled sections. A person skilled in the art could envision similar requirements and solutions using the basic idea of the embodiments of the present invention. It is contemplated that one, two, or more overmolds may be used depending on the geometry of the handpiece and the needs for sealing and/or connection.

The nosecone in some embodiments of the present invention may eliminate user installed O-rings, which are difficult to install for nurses or other users wearing gloves in the sterile field of an operating room. It provides the same critical sealing functionality without cumbersome installation. It eliminates the possibility to install O-rings incorrectly or in the wrong place, and eliminates the problem of assembling the wrong O-rings to surgical tip packs. It also enables a low cost disposable component created in two actuations of a single molding tool, and enhances ability to provide a clean and sterilized component to the operating room. The nosecone in some embodiments of the present invention improves ease of use in assembly, and provides easy and flawless assembly while maintaining functionality. It also enables a clean and sterile component for patient protection in the operating room.

While several inventive embodiments have been described and illustrated herein, those of ordinary skill in the art will readily envision a variety of other means and/or structures for performing the function and/or obtaining the results and/or one or more of the advantages described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. It is, therefore, to be understood that the foregoing embodiments are presented by way of example only and that, within the scope of the appended claims, inventive embodiments may be practiced otherwise than as specifically described.

All definitions, as defined and used herein, should be understood to control over dictionary definitions, definitions in documents cited herein, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of' shall be closed or semi-closed transitional phrases, respectively, as set forth in the United States Patent Office Manual of Patent Examining Procedures, Section 2111.03.

## Claims

1. A connection apparatus for attachments of members of a medical device, comprising: a first member (40) having a first body (411) and a first connecting section (412), the first connecting section (412) having a first end face (413) and an inner surface (414);
a second member (50) having a second body (57), a second connecting section (52) and a shoulder (54) at the junction between the second body (57) and the second connecting section (52), the second connecting section (52) having a second end face (58) and an outer surface (59) shaped for telescoping into the first connecting section (412), the outer surface (59) having a groove (56) extending from the second end face (58) towards the shoulder (541);
wherein the first member (40) has a nub (43) on the inner surface (414) extending radially inward for engaging the groove (56); and **characterised in that**
the first member (40) has an end overmold portion (42) at least partially covering the first end face (413) and positioned to bear against the shoulder (54), whereby the end overmold portion (42) is compressed between the first body (411) and the second body (57).

2. A connection apparatus as in claim 1, wherein the first member has more than one nub on the inner surface arranged radially about the first connecting section, and the second member has more than one groove on the outer surface arranged radially about the second connecting section, and each nub is positioned to engage with each groove.

3. A connection apparatus as in one of claims 1-2, wherein the end overmold portion is made of a thermoplastic elastomer.

4. A connection apparatus as in one of clams 1-3, wherein the first member is a nosecone and the second member is a surgical handpiece housing.

5. A connection apparatus as in one of claims 1-4, wherein the groove is J-shaped.

6. A connection apparatus as in one of claims 1-5, wherein the nub is sphere-shaped.

7. A connection apparatus as in one of claims 1-6, further comprising an internal overmold portion positioned radially about the inner surface of the first member.

8. A method for attaching members of a medical device, comprising:
providing a first member (40) having a first body (411) and a first connection section (412), the first connecting section (412) having a first end face (413), an inner surface (414), a nub (43) on the inner surface (414) extending radially inward, and an end overmold portion (42) at least partially covering the first end face (413);
providing a second member (50) having a second body (57), a second connecting section (52) and a shoulder (54) at the junction between the second body (57) and the second connecting section (52), the second connecting section (52) having a second end face (58) and an outer surface (59), the outer surface (59) having a groove (56) extending from the second end face (58) towards the shoulder (54);
telescoping the second connecting section (52) into the first connection section (412);
locating the groove (56) in the outer surface (59) of the second connecting section (52);
positioning the nub (43) to engage the groove (56) and moving the nub (43) in the groove (56) towards the shoulder (54) until the end overmold portion (42) contacts the shoulder (54) whereby the end overmold portion (42) is compressed between the first body (41) and the second body (57).

9. A method as in claim 8, further comprising:
providing more than one nub on the inner surface radially about the first connecting section;
providing more than one groove on the outer surface arranged radially about the second connecting section, wherein each nub is positioned to engage with each groove; and
positioning each nub to engage each groove; and
moving each nub in each groove towards the shoulder until the end overmold portion contacts the shoulder.

10. A method as in one of claims 8-9, wherein the overmold portion is made of a thermoplastic elastomer.

11. A method as in one of claims 8-10, wherein the first member is a nosecone and the second member is a surgical handpiece housing.

12. A method as in one of claims 8-11, wherein the groove is J-shaped.

13. A method as in one of claims 8-12, wherein the nub is sphere-shaped.

14. A method as in one of claims 8-13, further comprising an internal overmold portion positioned radially about the inner surface of the first member.

## Patentansprüche

1. Verbindungsvorrichtung für Anbringungen von Elementen eines medizinischen Geräts, die Folgendes umfasst:
ein erstes Element (40), das einen ersten Körper (411) und einen ersten Verbindungsabschnitt (412) aufweist, wobei der erste Verbindungsabschnitt (412) eine erste Endseite (413) und eine Innenoberfläche (414) aufweist;
ein zweites Element (50), das einen zweiten Körper (57), einen zweiten Verbindungsabschnitt (52) und einen Absatz (54) an der Anschlussstelle zwischen dem zweiten Körper (57) und dem zweiten Verbindungsabschnitt (52) aufweist, wobei der zweite Verbindungsabschnitt (52) eine zweite Endseite (58) und eine Außenoberfläche (59), die zum Zusammenschieben in den ersten Verbindungsabschnitt (412) geformt ist, aufweist, wobei die Außenoberfläche (59) eine Rille (56) aufweist, die sich von der zweiten Endseite (58) in Richtung des Absatzes (541) erstreckt;
wobei das erste Element (40) eine Noppe (43) auf der Innenoberfläche (414) aufweist, die sich radial nach innen zum Eingreifen in die Rille (56) erstreckt; und **dadurch gekennzeichnet ist, dass**
das erste Element (40) einen Endüberformteil (42) aufweist, der zumindest teilweise die erste Endseite (413) abdeckt und zum Anliegen gegen den Absatz (54) positioniert ist, wodurch der Endüberformteil (42) zwischen dem ersten Körper (411) und dem zweiten Körper (57) komprimiert wird.

2. Verbindungsvorrichtung nach Anspruch 1, wobei das erste Element mehr als eine Noppe auf der Innenoberfläche aufweist, die radial um den ersten Verbindungsabschnitt angeordnet sind, und das zweite Element mehr als eine Rille auf der Außenoberfläche aufweist, die radial um den zweiten Verbindungsabschnitt angeordnet sind, und jede Noppe zum Eingreifen in jede Rille positioniert ist.

3. Verbindungsvorrichtung nach einem der Ansprüche 1-2, wobei der Endüberformteil aus einem thermoplastischen Elastomer hergestellt ist.

4. Verbindungsvorrichtung nach einem der Ansprüche 1-3, wobei das erste Element ein Nasenkonus ist und das zweite Element ein chirurgisches Handstückgehäuse ist.

5. Verbindungsvorrichtung nach einem der Ansprüche 1-4, wobei die Rille J-förmig ist.

6. Verbindungsvorrichtung nach einem der Ansprüche 1-5, wobei die Noppe kugelförmig ist.

7. Verbindungsvorrichtung nach einem der Ansprüche 1-6, die weiter einen inneren Überformteil umfasst, der radial um die Innenoberfläche des ersten Elements positioniert ist.

8. Verfahren zur Anbringung von Elementen eines medizinischen Geräts, das Folgendes umfasst:
Bereitstellen eines ersten Elements (40), das einen ersten Körper (411) und einen ersten Verbindungsabschnitt (412) aufweist, wobei der erste Verbindungsabschnitt (412) eine erste Endseite (413), eine Innenoberfläche (414), eine Noppe (43) auf der Innenoberfläche (414), die sich radial nach innen erstreckt, und einen Endüberformteil (42), der zumindest teilweise die erste Endseite (413) abdeckt, aufweist;
Bereitstellen eines zweiten Elements (50), das einen zweiten Körper (57), einen zweiten Verbindungsabschnitt (52) und einen Absatz (54) an der Anschlussstelle zwischen dem zweiten Körper (57) und dem zweiten Verbindungsabschnitt (52) aufweist, wobei der zweite Verbindungsabschnitt (52) eine zweite Endseite (58) und eine Außenoberfläche (59) aufweist, wobei die Außenoberfläche (59) eine Rille (56) aufweist, die sich von der zweiten Endseite (58) in Richtung des Absatzes (54) erstreckt;
Zusammenschieben des zweiten Verbindungsabschnitts (52) in den ersten Verbindungsabschnitt (412);
Lokalisieren der Rille (56) in der Außenoberfläche (59) des zweiten Verbindungsabschnitts (52);
Positionieren der Noppe (43) zum Eingreifen in die Rille (56) und Bewegen der Noppe (43) in der Rille (56) in Richtung des Absatzes (54) bis der Endüberformteil (42) den Absatz (54) berührt, wodurch der Endüberformteil (42) zwischen dem ersten Körper (41) und dem zweiten Körper (57) komprimiert wird.

9. Verfahren nach Anspruch 8, das weiter Folgendes umfasst:
Bereitstellen von mehr als einer Noppe auf der Innenoberfläche radial um den ersten Verbindungsabschnitt;
Bereitstellen von mehr als einer Rille auf der Außenoberfläche, die radial um den zweiten Verbindungsabschnitt angeordnet sind, wobei jede Noppe zum Eingreifen in jede Rille positioniert ist; und
Positionieren von jeder Noppe zum Eingreifen in jede Rille; und
Bewegen von jeder Noppe in jede Rille in Richtung des Absatzes bis der Endüberformteil den Absatz berührt.

10. Verfahren nach einem der Ansprüche 8-9, wobei der Endüberformteil aus einem thermoplastischen Elastomer hergestellt ist.

11. Verfahren nach einem der Ansprüche 8-10, wobei das erste Element ein Nasenkonus ist und das zweite Element ein chirurgisches Handstückgehäuse ist.

12. Verfahren nach einem der Ansprüche 8-11, wobei die Rille J-förmig ist.

13. Verfahren nach einem der Ansprüche 8-12, wobei die Noppe kugelförmig ist.

14. Verfahren nach einem der Ansprüche 8-13, das weiter einen inneren Überformteil umfasst, der radial um die Innenoberfläche des ersten Elements positioniert ist.

## Revendications

1. Appareil de raccordement pour fixations de membres d'un dispositif médical, comprenant :
un premier membre (40) ayant un premier corps (411) et une première section de raccordement (412), la première section de raccordement (412) ayant une première face d'extrémité (413) et une surface interne (414) ;
un deuxième membre (50) ayant un deuxième corps (57), une deuxième section de raccordement (52) et un épaulement (54) à la jonction entre le deuxième corps (57) et la deuxième section de raccordement (52), la deuxième section de raccordement (52) ayant une deuxième face d'extrémité (58) et une surface externe (59) mise en forme pour se télescoper dans la première section de raccordement (412), la surface externe (59) ayant une rainure (56) s'étendant de la deuxième surface d'extrémité (58) vers l'épaulement (541);
dans lequel le premier membre (40) a une petite bosse (43) sur la surface interne (414) s'étendant radialement vers l'intérieur pour engager la rainure (56) ; et **caractérisé en ce que**
le premier membre (40) a une partie de surmoulure d'extrémité (42) recouvrant au moins partiellement la première face d'extrémité (413) et positionnée pour appuyer contre l'épaulement (54), en vertu de quoi la partie de surmoulure d'extrémité (42) est comprimée entre le premier corps (411) et le deuxième corps (57).

2. Appareil de raccordement selon la revendication 1, dans lequel le premier membre a plus d'une petite bosse sur la surface interne arrangée radialement autour de la première section de raccordement, et le deuxième membre a plus d'une rainure sur la surface externe arrangée radialement autour de la deuxième partie de raccordement, et chaque petite bosse est positionnée pour engager chaque rainure.

3. Appareil de raccordement selon l'une quelconque des revendications 1-2, dans lequel la partie de surmoulure d'extrémité est fabriquée en élastomère thermoplastique.

4. Appareil de raccordement selon l'une quelconque des revendications 1-3, dans lequel le premier membre est un cône de nez et le deuxième membre est un boîtier de pièce à main chirurgicale.

5. Appareil de raccordement selon l'une quelconque des revendications 1-4, dans lequel la rainure est en forme de J.

6. Appareil de raccordement selon l'une quelconque des revendications 1-5, dans lequel la petite bosse est en forme de sphère.

7. Appareil de raccordement selon l'une quelconque des revendications 1-6, comprenant en outre une partie de surmoulure interne positionnée radialement autour de la surface interne du premier membre.

8. Procédé de fixation de membres d'un dispositif médical, comprenant :
fournir un premier membre (40) ayant un premier corps (411) et une première section de raccordement (412), la première section de raccordement (412) ayant une première face d'extrémité (413), une surface interne (414), une petite bosse (43) sur la surface interne (414) s'étendant radialement vers l'intérieur, et une partie de surmoulure d'extrémité (42) recouvrant au moins partiellement la première face d'extrémité (413) ;
fournir un deuxième membre (50) ayant un deuxième corps (57), une deuxième section de raccordement (52) et un épaulement (54) à la jonction entre le deuxième corps (57) et la deuxième section de raccordement (52), la deuxième section de raccordement (52) ayant une deuxième face d'extrémité (58) et une surface externe (59), la surface externe (59) ayant une rainure (56) s'étendant de la deuxième face d'extrémité (58) vers l'épaulement (54) ;
télescoper la deuxième section de raccordement (52) dans la première section de raccordement (412) ;
placer la rainure (56) dans la surface externe (59) de la deuxième section de raccordement (52) ;
positionner la petite bosse (43) pour engager la rainure (56) et déplacer la petite bosse (43) dans la rainure (56) vers l'épaulement (54) jusqu'à ce que la partie de surmoulure d'extrémité (42) entre en contact avec l'épaulement (54) en vertu de quoi la partie de surmoulure d'extrémité (42) est comprimée entre le premier corps (41) et le deuxième corps (57).

9. Procédé selon la revendication 8, comprenant en outre :
fournir plus d'une petite bosse sur la surface interne radialement autour de la première section de raccordement ;
fournir plus d'une rainure sur la surface externe arrangée radialement autour de la deuxième section de raccordement, dans lequel chaque petite bosse est positionnée pour engager chaque rainure ; et
positionner chaque petite bosse pour engager chaque rainure ; et
déplacer chaque petite bosse dans chaque rainure vers l'épaulement jusqu'à ce que la partie de surmoulure d'extrémité entre en contact avec l'épaulement.

10. Procédé selon l'une quelconque des revendications 8-9, dans lequel la partie de surmoulure est fabriquée en élastomère thermoplastique.

11. Procédé selon l'une quelconque des revendications 8-10, dans lequel le premier membre est un cône de nez et le deuxième membre est un boîtier de pièce à main chirurgicale.

12. Procédé selon l'une quelconque des revendications 8-11, dans lequel la rainure est en forme de J.

13. Procédé selon l'une quelconque des revendications 8-12, dans lequel la petite bosse est en forme de sphère.

14. Procédé selon l'une quelconque des revendications 8-13, comprenant en outre une partie de surmoulure interne positionnée radialement autour de la surface interne du premier membre.
